# EUROPEAN PATENT APPLICATION

(11) **EP 1 577 083 A1**
(43) Date of publication of application: **21.09.2005**
(21) Application number: 03769957.6
(22) Date of filing: 29.10.2003
(51) Int. Cl.: B32B 5/26, A61L 27/14, A61L 27/56, A61L 27/38

(54) **BIODEGRADABLE SUBSTRATE, PROSTHETIC MATERIAL FOR TISSUE REGENERATION AND CULTURED TISSUE**

(30) Priority: 31.10.2002 JP 2002319169
(71) Applicant: NIPRO CORPORATION, Osaka 531 (JP)
(72) Inventor: MORINAGA, Yukihiro, c/o NIPRO, Osaka-shi, Osaka 531-8510 (JP); MATSUDA, Kazuhisa, c/o Nipro, Osaka-shi, Osaka 531-8510 (JP); KAMIMURA, Ryosuke, c/o Nipro, Osaka-shi, Osaka 531-8510 (JP)
(74) Representative: Albrecht, Thomas
(86) International application number: PCT/JP2003/013847
(87) International publication number: WO 2004/039578

(57) **Abstract**

A biodegradable substrate usable as a prosthetic material for medicinal use (for example, tissue regeneration) or a cell culture substrate which is highly compatible with cells, in particular, facilitates the invasion of cells into the inside of the substrate and has a thick structure similar to woven fabrics without resort to any troublesome techniques such as lamination or weaving/knitting. The biodegradable substrate is obtained by stitching a biodegradable nonwoven fabric with a biodegradable threadlike article. As the nonwoven fabric, use is made of, for example, a material wherein first, and second layers, each having filaments of a threadlike article made of collagen located in parallel, are laminated and adhered to each other so that the alignment direction of the filaments of the threadlike article in the first layer and that of the second layer are arranged at a certain angle. This nonwoven fabric is stitched with a threadlike article made of collagen. It is also possible that a filmy material having been treated with a biodegradable binder such as collagen or gelatin is piled on the nonwoven fabric and stitched.

## Description

### TECHNICAL FIELD

The present invention relates to a biodegradable substrate, i.e., a substrate for medical use, which is made of a biodegradable material typified by collagen. In particular, the present invention relates to a substrate for medical use, which can be used for various prosthetic materials including a substrate used in cell culture for transplantation in regenerative medicine; a substrate for medical use, which can be used for various filling materials or prosthetic materials, which promote regenerative induction by making up defects in living bodies; and a substrate for medical use, which can be used for various carriers including a sustained-release DDS carrier and a carrier for genetic therapy. Furthermore, the present invention relates to these uses, in particular, a prosthetic material for tissue regeneration and a cultured tissue.

### BACKGROUND ART

In recent years, various proposals have been made to employ woven and nonwoven fabrics made of biodegradable materials such as collagen, gelatin, and hyaluronic acid as substrates for medical use or to employ sterically-shaped textile and knitting fabrics made of collagen threads as substrates for medical use (see, for example, JP 09-47502 A (pages 2-3), Japanese Patent No. 3086822B, JP 2000-93497 A, JP 2000-210376 A, JP 2000-271207 A, and JP 09-510639 T2).

These substrates for medical use are preferable in that they can provide comparatively large surface areas as well as being excellent in affinity to cells, such as material permeability and the invasion of cells into the substrate. In particular, since these substrates for medical use are made of biodegradable materials, they have advantages in that they do not cause any foreign-substance reaction in the living body and can be directly embedded therein for a long time.

Nonwoven fabrics made of collagen fibers or gelatin fibers are usually prepared by a wet-type paper-making method. In this case, however, the nonwoven fabrics thus obtained are generally fragile. In addition, it is difficult to uniformly disperse a slurried thread, and thus it is difficult to equalize the strength or thickness of the nonwoven fabric.

Also, a nonwoven fabric itself is a flat article. For making a three-dimensional tissue having a certain thickness, there is a need to laminate thinly-formed tissues. On this account, a complicated procedure of using a previously-laminated substrate, laminating a cultured tissue, or the like is required.

Furthermore, for increasing the strength of a nonwoven fabric, a dilute solution of collagen or gelatin has been sprayed on the nonwoven fabric thus obtained to form a filmy material. However, this work is also complicated and lowers the degrees of material permeability and the invasion of cells into the substrate. Therefore, the resulting substrate is insufficient to be used as a substrate for cell culture.

In this regard, a three-dimensional substrate prepared by weaving and knitting collagen threads as described in JP 09-510639 T2 is a preferable substrate for cell culture on account of involving none of the disadvantages described above while having good affinity to cells and excellent strength.

However, a woven fabric or the like having a three-dimensional configuration has threads extending alongmultiple axial directions, and collagen threads should be woven and knitted in a three-dimensional structure. Therefore, such a woven fabric is fabricated with technical difficulties.

Besides, a single thread (collagen thread) having strength enough to be woven and knitted is very difficult to produce. Even if a single thread (collagen thread) can be produced, it is difficult to produce one having strength enough to allow the thread to be woven and knitted. Therefore, in the Example of JP 09-510639 T2, a double-ply yarn is obtained at first.

The present invention has been made to solve the above-mentioned problems of the conventional techniques. An object of the present invention is to provide a biodegradable substrate for medical use, which has a structure analogous to a thick woven fabric or a knitted fabric without requiring a technically-difficult process of lamination, weaving/knitting, or the like while improving affinity to cells, particularly facilitating introduction of cells into the inside of the substrate.

### DISCLOSURE OF THE INVENTION

A biodegradable substrate of the present invention has such a feature that a biodegradable nonwoven fabric is stitched with a biodegradable thread.

In addition, the biodegradable substrate of the present invention is characterized in that a biodegradable nonwoven fabric and a biodegradable filmy material are piled on one another and both of them are stitched with a biodegradable thread. For the biodegradable substrate, as the biodegradable filmy material one having the form of a film or a sponge is preferably used.

In the present invention, for example, as the biodegradable nonwoven fabric, use may be made of a laminated product in which a first layer and a second layer, each of which is constructed of a plurality of biodegradable threads arranged in parallel, are piled on one another such that the directions of the threads of the first and second layers are arranged at an angle and are bonded to each other.

In addition, there may be used a biodegradable nonwoven fabric that includes a laminated product in which a third layer having a plurality of additional biodegradable threads arranged in parallel is laminated on the first or second layer such that the arranging direction of the threads arranged in the third layer is at an angle with the arranging direction of the threads arranged in the layer adjacent to the third layer and these layers are bonded to each other.

In each of these cases, the acute angle between the arranging directions of the threads of the first layer and the second layer, and/or the acute angle between the arranging directions of the threads of the third layer and the layer adjacent to the third layer are/is preferably about 20° or less.

Furthermore, the acute angle between the arranging directions of the threads of the first layer and the second layer, and/or the acute angle between the arranging directions of the threads of the third layer and the layer adjacent to the third layer are/is preferably about 70 to 90°.

As the biodegradable nonwoven fabric, use may be made of a plurality of the laminated products, where each laminated product is composed of a plurality of layers having threads arranged therein and the laminated products are piled on one another such that the arranging directions of the threads of the layers adjacent to each other at an interface between the laminated products are at an angle with each other and are bonded to each other.

Of these biodegradable nonwoven fabrics, an acute angle which is formed by each of the threads arranged in each layer is preferably about 0 to 5°. A space between the threads arranged in each layer is preferably about 0 to 40 mm.

In the present invention, stitches may be in a dotted pattern. The intervals of stitches are desirably about 0.1 mm to 100 mm.

Furthermore, in the present invention, the surface of the biodegradable nonwoven fabric and/or the surface of the biodegradable thread are/is preferably coated with a biodegradable material. The biodegradable material is characteristically made of one or more of a material selected from the group consisting of collagen, gelatin, PLA, PLA derivatives, PGA, PGA derivatives and copolymers formed from two or more of PLA, PLA derivatives, PGA and PGA derivatives.

The biodegradable nonwoven fabric and/or the biodegradable thread used in the present invention is preferably made of one or more of a material selected from the group consisting of collagen, gelatin, PLA, PLA derivatives, PGA, PGA derivatives and copolymers formed from two or more of PLA, PLA derivatives, PGA and PGA derivatives.

The prosthetic material for tissue regeneration of the present invention is characterized by a prosthetic material for tissue regeneration for filling a defective portion in a biological tissue, comprising a biodegradable substrate of the present invention as mentioned above.

The culture tissue of the present invention is characterized in that tissue cells in the living body are adhered, while maintaining their function, to the biodegradable substrate of the present invention.

### BRIEF DESCRIPTION OF THE DRAWINGS

Fig. 1 is an explanatory diagram illustrating one example of a method of manufacturing a biodegradable nonwoven fabric used in the present invention.
Fig. 2 is an explanatory diagram illustrating a method of manufacturing a biodegradable substrate according to an embodiment of the present invention.
Fig. 3 is an explanatory diagram illustrating a method of manufacturing a biodegradable substrate according to another embodiment of the present invention.
Fig. 4 is a photograph representing a state in which fibroblasts adhere, while maintaining their function, to the biodegradable substrate of the present invention by culturing.
Fig. 5 is a photograph representing a state in which human chondrocytes adhere, while maintaining their function, to the biodegradable substrate of the present invention by culturing.

### BEST MODE FOR CARRYING OUT THE INVENTION

A biodegradable substrate of the present invention is characterized as comprising a biodegradable nonwoven fabric, where the biodegradable nonwoven fabric is stitched using a biodegradable nonwoven article. The term "biodegradable" as used in the present invention means that, when placed in a human or animal living body, a substance will be fused with a human or animal tissue or completely decomposed in the living body, and the substance to be used is any material adaptive to the living body. Examples of the biodegradable nonwoven fabric or biodegradable thread used in the present invention include living body-derived biomaterials such as collagen and gelatin, biodegradable synthetic materials such as PLA (polylactic acid), PLA derivatives, PGA (polyglycolic acid), PGA derivatives and copolymers of two or more selected from PLA, PLA derivatives, PGA and PGA derivatives. Of these, collagen is preferable in terms of cytotropic and biocompatible properties. Furthermore, most preferable is atelocollagen which has been subjected to a treatment for removing telopeptide which is an antigenic determinant of collagen, and to a solubilization treatment at the same time. In general, the original sources of collagen include, but are not particularly limited to, cows, pigs, birds, fish, rabbits, sheep, mice, and human beings. The collagen can be obtained from skins, tendons, bones, cartilages, and organs of these animal species by any of various extraction methods known in the art. In addition, collagen is not limited to any of those which can be classified into types I, II, and III. Of these, however, the type I is particularly preferable in terms of handling.

The biodegradable nonwoven fabric used in the present invention is any of various nonwoven fabrics which have been known in the art and those described in Patent Document 1 or 2 described above are exemplified. As will be described hereinafter, a nonwoven fabric including a laminated product structure in which first and second layers each having a plurality of biodegradable threads arranged in parallel are piled on one another is more preferably used.

Here, the layer having a plurality of biodegradable threads arranged in parallel means a layer in which threads are linearly arranged on the same plane at substantially regular intervals. An angle between the threads arranged on the same layer is about 0 to 5°, preferably 0°. In other words, the term "parallel" as used in the present invention means the range defined by this angle, but does not precisely mean "parallel". The distance between the threads on the same layer is generally in the range of about 0 to 40 mm, preferably about 0 to 10 mm, more preferably about 0 to 1 mm.

The term "lamination" indicates that the arranging directions of the threads of the first layer and the second layer are at an angle and the threads are piled on one another while being bonded to each other, and the acute angle between the directions in which the thread is arranged in the first layer and the thread is arranged in the second layer is not 0°. The phrase "first and second layers are piled on one another" means such a state that the first and second layers are in surface contact with each other.

The biodegradable nonwoven fabric suitably used in the present invention may be one having a laminated product constructed of at least two layers as described above, wherein a third layer having a plurality of such biodegradable threads arranged therein in parallel is further laminated such that the arranging directions of the threads of the first or second layer and the third layer extend at an angle with each other to form a laminated product of three layers bonded to one another. Furthermore, the biodegradable nonwoven fabric may include a laminated product constructed of four layers, where, on both sides of the laminated product composed of the first and second layers, the layers having biodegradable threads arranged therein in parallel are similarly laminated. Alternatively, it may include a laminated product constructed of five or more layers, in which an additional layer having a plurality of threads arranged in parallel is similarly laminated. In this way, the nonwoven fabric is one including two or more layers piled on one another such that the arranging directions of the threads of the layers adjacent to each other are at an angle with each other.

In the present invention, the term "layers adjacent to each other" means a state in which the layers having threads arranged therein are in surface contact with each other, and the "layers adjacent to each other" means upper and lower layers laminated in surface contact with each other. Angles between the threads in a layer to be laminated as a third layer and the intervals between those threads are preferably within the ranges described above, respectively.

In these nonwoven fabrics, the acute angle between the arranging directions of the threads of the first and second layers may be any other angle than 0°, preferably 20° or less, more preferably 10° or less. In addition, the arranging direction of the biodegradable thread of one layer is at an angle with the arranging direction of the layer adjacent thereto. The thread arranging directions of the layers which are not adjacent to each other do not always need to form any angle therebetween, that is, the angle therebetween may be 0°. For example, in the laminated product composed of three layers, when the third layer is laminated on the second layer, the arranging directions of the threads of the first and second layers should be at an angle with each other and also those of the second and third layers should be at an angle with each other. However, the arranging directions of the threads of the first and third layers may be at an angle with each other or may be at an angle of 0°. The angle between the arranging directions of the threads in the third and second layers is preferably about 20° or less, more preferably 10° or less. Note that the case in which the arranging directions of the threads of the first and third layers are at an angle of 0° with each other is the case in which the arranging direction of the threads of the third layer is at an acute angle of -20° with that of the second layer when the arranging direction of the thread of the second layer is at an acute angle of 20° with that of the first layer. Of course, there is no trouble even if the arranging direction of the thread of the third layer is additionally at an angle of 20° with that of the second layer, i.e., at an acute angle of 40° with that of the first layer.

The nonwoven fabric of the present invention may have an acute angle of preferably about 70° to 90°, more preferably at about 80° to 90° between the arranging directions of the threads of the first and second layers. In this case as well, the arranging directions of the biodegradable threads are at an angle with each other in the layers adjacent to each other, while the arranging directions of the threads of the layers which are not adjacent to each other are not necessarily at an angle with each other, so that they can be at an angle of 0° with each other. Furthermore, when the third layer is laminated, the acute angle between the arranging direction of the thread of the layer adjacent to the third layer and that of the third layer is preferably about 70° to 90°, more preferably 80 to 90°. By piling the first, second, and third layers at angles within the range of the angles described above, a nonwoven fabric in which threads arranged in parallel in the respective layers are laminated almost perpendicularly to each other can be obtained.

The laminated product including the three or more layers may be a laminated product in which the arranging directions of the threads to be laminated are kept at a constant angle or may be a laminated product in which the angle between the arranging directions of the threads may be set at random. The former may be, for example, a laminated product in which a first layer and a second layer are piled one after the other, where an acute angle between the arranging directions of the threads of a third layer laminated on the second layer and the first layer is 0°, in such a manner that an acute angle between the arranging directions of threads of the first and second layers is at a constant angle of 20° or less. The latter may be, for example, a laminated product in which first, second, third, and other subsequent layers are piled on one another at random while angles between the arranging directions of threads of the layers adjacent to each other vary within the range of 20° or less.

Furthermore, the nonwoven fabric used in the present invention may include a laminated product formed by piling a plurality of laminated products constructed of a plurality of layers as described above. For instance, there is a case in which the third layer forms a second laminated product different from a laminated product formed by piling the first and second layers one after the other. In this case, the arranging directions of the threads of the layers adjacent to each other at the interface between the laminated products adjacent to each other are at an angle with each other. In case where the third layer forms the second laminated product, the arranging direction of the thread of the second layer provided as the top layer of the first laminated product and the arranging direction of the thread of the third layer provided as the bottom layer of the second laminated product are at an angle with each other. In addition, the second laminated product may be a laminated product in which a plurality of third layers are piled on one another and, similar to the first laminated product, the first and second layers, each of which includes a plurality of biodegradable threads arranged in parallel, are piled on one another such that the arranging directions of the threads of the first and second layers can be at an angle and they are bonded to each other. Three or more layers may be piled on one another and bonded to each other.

For the nonwoven fabric in which a plurality of laminated products are piled up one another, an acute angle between the arranging directions of the threads of the layers adjacent to each other at the interface of the laminated products adjacent to each other may be any other angle than 0°, preferably 70° to 90°, more preferably 80° to 90° . For instance, a nonwoven fabric constructed of threads arranged lengthwise and breadthwise can be obtained when laminated products in which an acute angle between the arranging directions of the threads is 20° or less are piled on one another at an angle of 70° or more. Further, if they are piled on one another at an angle of 20° or less, the same effect as that of one prepared by piling the first and second layers one after the other in larger numbers can be obtained. In case where three or more laminated products are piled, the angle at the interface of the laminated products adjacent to each other may be kept constant or may be at random. As the former, for example, there is a laminated product in which a first laminated product and a second laminated product are piled on one another at a constant acute angle of about 70° to 90°.

In the above cases, a nonwoven fabric can be fabricated by bonding the threads of the layers adjacent to each other at contact portions. For instance, when biodegradable threads used are threads prepared by a wet spinning method described later, which are not dried (in wet condition), they can be bonded by subjecting them to a drying process after lamination. In case of the biodegradable threads which were subjected to drying, crosslinking treatment and so forth after spinning, adhesion can be carried out such that a biodegradable material, e.g., a biodegradable polymer is applied on a nonwoven fabric by spraying or impregnation after lamination and is then dried.

The thread used in the nonwoven fabric including the above laminated product is not particularly limited as far as it has flexibility enough to be wound up like a general thread. One having higher strength is preferable, but as disclosed in JP 9-510639 T2, strength enough to bear the processing with a weaving machine or a knitting machine is not needed. In other words, as described below, the thread has only to be wound around a plate member.

For instance, such threads include collagen threads prepared by spinning solubilized collagen provided as a spinning stock solution. Spinning a solubilized collagen solution as a spinning stock solution means that the collagen solution is used as a raw material and spun by any of various spinning methods known in the art such as a wet-type spinning method (see, for example, Patent Documents 3 to 5 above, and JP 6-228505 A, and JP 6-228506 A).

The term "solubilized collagen" means collagen modified so as to be soluble in a solvent, such as acid-solubilized collagen, alkali-solubilized collagen, enzyme-solubilized collagen and neutral-solubilized collagen. In particular, preferred is atelocollagen which has been subjected to treatment for removing a telopeptide which is an antigenic determinant of the collagen simultaneously with a solubilization treatment. These solubilization methods are described in JP 46-15003 B, JP 43-259839 B, JP 43-27513 B, and so on. Note that the collagen may be originated from, but not particularly limited to, an extraction product of any of the animal species or portions described above.

A solvent used for a solubilized collagen solution is not particularly limited as far as it makes collagen soluble. Representative solvents include dilute acid solutions such as hydrochloric acid, acetic acid and nitric acid, mixtures of water and hydrophilic organic solvents such as ethanol, methanol and acetone. Each of them may be used alone or two or more of them may be used in combination at any ratio. Of these, preferable is water.

The concentration of collagen in the collagen solution is not particularly limited as far as it allows spinning, but preferably in the range of about 4 to 10% by weight, more preferably about 5 to 7% by weight.

The diameter of the collagen thread is not particularly limited. The diameter of the collagen thread is preferably about 5 µm to 1.5 mm, and more preferably about 10 to 200 µm.

In case where the collagen thread is spun by a wet spinning method, a collagen thread used for a nonwoven fabric may be one in a wet condition prepared by the wet spinning method, which is not subjected to drying, or may be one obtained by subjecting the article to drying, a crosslinking treatment and so forth after spinning.

Examples of the wet spinning method for preparing the collagen thread include a method using a hydrophilic organic solvent and a method using a crosslinking agent. Of these, a collagen thread spun by the method using the crosslinking agent is preferably used.

When the wet spinning is performed using the hydrophilic organic solvent, in general, a collagen solution is continuously discharged from a nozzle into a bath filled with a desolvating agent such as the hydrophilic organic solvent, followed by dehydration and solidification to obtain a collagen thread. Examples of the hydrophilic organic solvent include alcohols having 1 to 6 carbon atoms, such as ethanol, methanol and isopropanol, and ketones such as acetone and methylethylketone. Each of these solvents may be used alone, or two or more of them may be used in combination at any ratio. Of these, a preferable solvent is ethanol. The water content of the hydrophilic organic solvent is generally about 50% by volume or less, preferably about 30% by volume or less. The process of spinning a collagen solution (dehydration and solidification) using the hydrophilic organic solvent is generally carried out at room temperature to about 42°C. A processing time of a sequence of dehydration and solidification is from about 4 to 5 seconds to 5 hours.

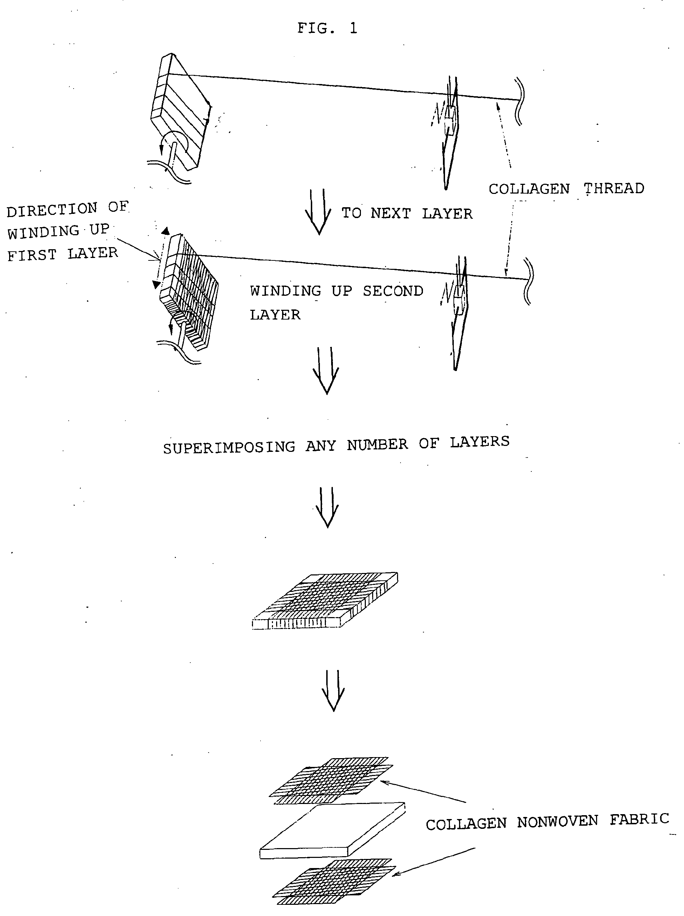

Themethod of manufacturing the nonwoven fabric described above is illustrated in Fig. 1. In this method, a plate member 1 is turned around a fixed rotation axis 2 and the collagen thread obtained above is then wound up to pile a plurality of layers in which a plurality of threads are arranged in parallel to forma first laminated product. Subsequently, a secondary laminated product is formed by further winding the collagen thread in parallel around a portion contacting with the first product such that the arranging directions of threads of layers adjacent to each other are at an angle with each other. Furthermore, in the illustrated example, the second laminated product is formed by shifting the rotation axis 2 of the plate member 1.

The plate member 1 is a member which itself turns or the like to wind up the collagen thread. The raw material thereof is not particularly limited as far as it is a material capable of maintaining a winding state without causing adhesion to the collagen thread. Preferably, however, it is a metal, a resin, or the like. More preferably, it is a stainless steel, a polyfluroethylene-based resin, or the like. The shape of the plate member 1 is not particularly limited as far as it allows a collagen thread to be wound up in at least two directions. Preferably, it is in the form of a plate or frame having at least three sides. More preferably, it is in the form of substantially a square-shaped plate or frame.

Turning the plate member 1 around the fixed rotation axis 2 means that the plate member 1 rotates around an axis horizontally penetrating through the surface of the plate member 1. In addition, shifting the rotation axis 2 of the plate member 1 refers to allowing the plate member 1 to rotate around an axis 2 passing through the plate member 1, which is different from the rotation axis 2, such that it rotates "around an axis parallel to another side of the plate member which intersects the rotation axis". The rotation axis 2 is shifted to allow the collagen thread to be wound up in another direction of the plate member 1. This operation is repeated to obtain a nonwoven fabric in which a plurality of laminated products including first layers and second layers having the above threads arranged in parallel are laminated. Furthermore, in Fig.1, reference numeral 3 denotes a reciprocating mechanism for allowing the thread to be moved reciprocately in the winding up direction.

A driving method for turning the plate member 1 is not particularly limited. However, it is preferable to be driven by a constant mechanical driving force. Furthermore, an operation for shifting the rotation axis 2 of the plate member 1 may be performed by hand or by a device or the like that automatically shifts the rotation axis 2. When a collagen nonwoven fabric is produced on an industrial scale, it is desirable to use an apparatus that automatically shifts the rotation axis 2 in a mechanical manner.

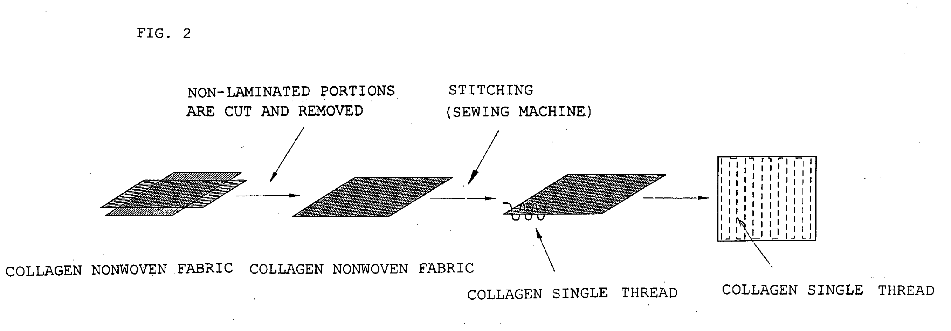

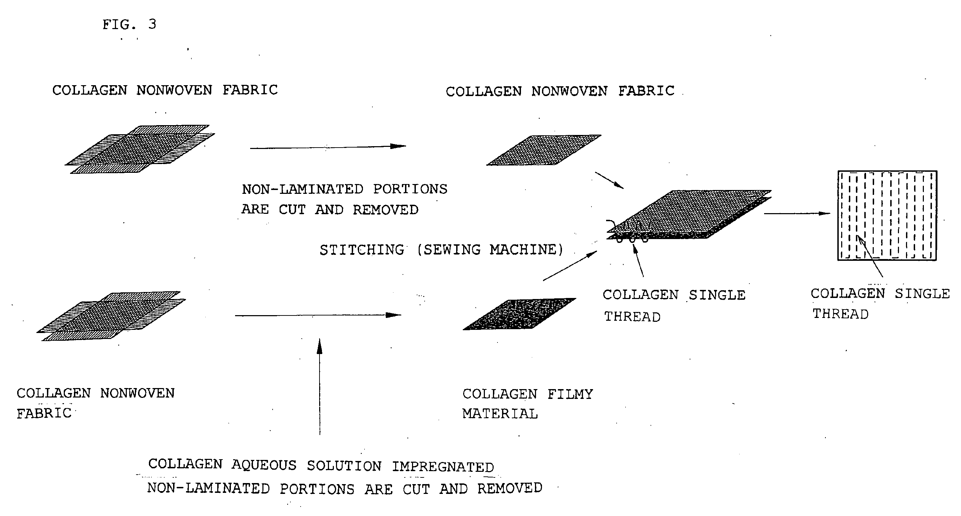

In general, when a collagen thread is wound up around the plate member 1 with a constant winding-up width, the rotation axis 2 of the plate member 1 is shifted after winding up the thread by a plurality of reciprocal movements on one side of the plate member 1. In other words, a layer (first layer) is formed by winding up the thread on one side of the plate member 1 in one direction and then another layer (second layer) is formed by winding up the thread on the same side of the plate member 1 in a reverse direction. Therefore, a laminated product including laminated first and second layers can be obtained by winding up by a reciprocating movement on the one side of the plate member 1. Therefore, a laminated product (first laminated product), in which a first layer and a second layer are respectively laminated according to the number of times of reciprocating movement can be obtained by several reciprocating movements. The acute angle between the arranging directions of threads when the thread is wound up forward and backward by a reciprocating movement is generally 20° or less, preferably about 10° or less . This angle is set as an acute angle between the arranging directions of threads of the first and second layers (or an acute angle between the arranging directions of threads of the third layer and the layer adjacent to the third layer) . Then, this angle can be adjusted by the size of the plate member 1, the number of rotations of the plate member 1, and the reciprocating speed of a reciprocating mechanism 3. In addition, even after shifting the rotation axis 2, the winding is performed similarly, and a second laminated product is obtained. However, the acute angle between the arranging directions of the threads before shifting the rotation axis 2 and after shifting the rotation axis 2 is generally about 70° to 90°, preferably about 80° to 90°. This angle is provided as an acute angle between the arranging directions of threads of the layers at an interface between the laminated products adjacent to each other. Of course, the rotation axis 2 of the plate member 1 may be shifted after every reciprocating movement. One side of the plate member 1 winds up in one direction and then the rotation axis 2 of the plate member 1 is shifted to allow the plate member 1 to wind up in another direction. Like the latter case, the acute angle between the arranging directions formed after winding up by shifting the rotation axis 2 after winding up in one direction corresponds to the acute angle between the arranging directions of the threads of the first and second layers (or the acute angle between the arranging directions of the threads of the third layer and the layer adjacent to the third layer). In this case, the acute angle is preferably about 70° to 90°. Laminated products obtained by such a reciprocating movement can be piled on one another to form a laminate of several layers. Note that a thin layer portion located on the peripheral portion (portion corresponding to a so-called frame of the plate member 1) of the thus obtained laminated product is generally removed by cutting (see Figs. 2 and 3).

The collagen nonwoven fabric obtained by the method as described above may optionally be subjected to various kinds of known physical or chemical crosslinking treatment if necessary. The crosslinking treatment may be carried out at any stage. In other words, the nonwoven fabric may be formed using a thread which has been subjected to various crosslinking treatments. Alternatively, various crosslinking treatments may be performed after forming the nonwoven fabric. Examples of the physical crosslinking method include a gamma irradiation, an ultraviolet irradiation, an electron beam irradiation, a plasma irradiation, and a thermal dehydration reaction. On the other hand, examples of the chemical crosslinking method include a reaction with aldehyde derivatives such as dialdehyde and polyaldehyde, epoxy derivatives, carbodiimide derivatives or isocyanate derivatives, a tannin treatment, and a chromium treatment. Of these, the physical crosslinking treatment is preferably a thermal dehydration crosslinking treatment, and the chemical crosslinking treatment is preferably a crosslinking treatment with glutaraldehyde. Furthermore, the biodegradable nonwoven fabric obtained by the above method may be coated with a biodegradable material. Examples of the coating material include collagen, hyaluronic acid, and gelatin.

As an example of the method of coating with the coating material, there may be mentioned a binder treatment. The binder treatment means a treatment in which a nonwoven fabric is impregnated with a solution material and then dried by an appropriate drying method to reinforce bonding between the threads in the nonwoven fabric. A collagen nonwoven fabric is formed as a filmy material by the binder treatment to improve a physical strength more than an untreated nonwoven fabric. Thus, the strength for stitching can be also improved markedly.

However, in performing the binder treatment, in case where a collagen nonwoven fabric is not subjected to a crosslinking treatment, there is possibility that the nonwoven fabric itself dissolves in a solvent with which the nonwoven fabric is impregnated. Therefore, it is desirable to conduct crosslinking treatment beforehand. A collagen nonwoven fabric may be subjected to crosslinking treatment again after the coating treatment, if necessary. In addition to these treatments, various methods of reinforcing bonding between the threads in the collagen nonwoven fabric can be suitably used.

Furthermore, it is feasible to produce a nonwoven fabric after coating a thread which was subjected to crosslinking treatment, with a coating material in advance.

The nonwoven fabric may be subjected to a treatment for entwining threads in each layer with one another. As a treatment method, for example, there may be mentioned a treatment method by which threads of each of layers in a collagen nonwoven fabric, which are piled on one another, are complicatedly entwined with one another at random by a needle punch. By such a treatment, a collagen nonwoven fabric can be obtained in the form of a felt. The felt-like collagen nonwoven fabric may be subjected to a binder treatment, if required.

Also, a nonwoven fabric can be used, which is prepared by use of a biodegradable thread obtained from a biodegradable material such as gelatin, in addition to collagen, and the nonwoven fabric can be used, which is prepared by use of a biodegradable thread article obtained from a synthetic material as described above. These nonwoven fabrics can be produced by the known methods, for example, a method described in Patent Document 6 above as an example of the methods using gelatin.

The biodegradable substrate of the present invention is obtained by stitching the thus obtained nonwoven fabric with the biodegradable thread. In other words, if it is compared to woven fabric, the thread (hereinafter, referred to as "thread for stitching") fulfills a function as a warp thread for interweaving a two-dimensional woven fabric in the vertical direction. The thread for stitching fixes threads or fibers not interwoven which constitute the nonwoven fabric, preventing the threads or fibers from getting loose to increase the strength of the nonwoven fabric. Fig. 2 illustrates a state in which a biodegradable substrate is produced by stitching the nonwoven fabric.

The biodegradable thread for stitching is not particularly limited as far as it has flexibility like a normal thread and can be wound up. For example, the thread used for the manufacture of the biodegradable nonwoven fabric mentioned above can be employed as it is. In addition, the thread for stitching may be a single thread or a twisted thread. If it is a twisted thread, the number of threads to be twisted is, but is not limited to, preferably about 2 to 6. In addition, in case where it is a twisted thread, the above crosslinking treatment may be performed during the process of twisting or the thread may be subjected to the crosslinking treatment in advance and then subjected to the process of twisting.

The term "stitching" means that a nonwoven fabric is stitched using the above biodegradable single thread or twisted thread. For instance, any of stitching methods used in the industry of dress and their ornaments, such as straight stitch, wave stitch, half-back stitch, and blanket stitch can be adopted. Preferably, a nonwoven fabric can be stitched to such a degree that threads and fibers forming the nonwoven fabric cannot be loosened. The stitching may be performed by hand or using a machine such as a sewing machine. A stitching position may be any position depending on the size thereof and the purpose of use as far as it attains the object described above. It is preferable to stitch up at least the periphery of the biodegradable nonwoven fabric, more preferably, uniformly the whole area of the nonwoven fabric. In addition, when the whole area of the nonwoven fabric is stitched, stitching is performed so that stitching lines are aligned in the same direction or stitching lines traverse in the vertical or horizontal direction. Preferably, stitching is performed such that stitching lines can be traversed at an angle of about 90°.

The stitching may be not always continuously performed. Stitch points like those found in a cushion are allowable. Any number of stitches can be allowed. For instance, the stitches may be only provided on the central portion of the nonwoven fabric, but if the number of stitches is too small, warp threads and weft threads may become easily loose. Preferably, therefore, the stitches are uniformly dispersed.

The intervals (pitches) of the stitching or stitches are, but are not particularly limited to, preferably in the range of about 0.1 mm to 100 mm, more preferably in the range of about 1 mm to 10 mm. However, in case where a filmy nonwoven fabric is stitched or a filmy material described later and the biodegradable nonwoven fabric are combined together and then stitched, the strength of the filmy material may be decreased, since the number of stitching pores penetrated through the filmy material increases too much, and the pitches of the stitching or the intervals of the stitches are narrowed too much. Therefore, attention should be given to this possibility.

In the present invention, as described above, it is possible to not only stitch one piece of biodegradable nonwoven fabric, but also to pile two or three of the fabrics on one another to be stitched together. For instance, another biodegradable filmy material may be superimposed on the biodegradable nonwoven fabric as shown in Fig. 3 and then stitched together. Therefore, a substrate for medical application having a thickness almost equal to or higher than that of a woven fabric can be obtained. The thickness of the biodegradable nonwoven fabric is, but not particularly limited to, generally about several hundreds of µm to several mm, or several tens of mm or more in the case of a larger size. In addition, the thickness of the biodegradable nonwoven fabric varies depending on the use thereof and is, for example, practically about 5mm at most in the case of a cell-culture substrate as described later.

As is in the case of the biodegradable nonwoven fabric or the biodegradable thread described above, the biodegradable filmy materials to be superimposed include those made of living body-derived biomaterials such as collagen and gelatin, and biodegradable synthetic materials such as PLA (polylactic acid), PLA derivatives, PGA (polyglycolic acid), PGA derivatives, and copolymers of two or more selected from PLA, PLA derivatives, PGA, and PGA derivatives. Of these, collagen is preferable in terms of cytotropic and biocompatible properties. Furthermore, most preferable is atelocollagen which has been subjected to a treatment of removing telopeptide which is an antigenic determinant of collagen, simultaneously with a solubilization treatment. In general, the original sources of collagen include, but are not particularly limited to, cows, pigs, birds, fish, rabbits, sheep, mice, and human beings. The collagen can be obtained from skins, tendons, bones, cartilages, and organs of these animal species by any of various extraction methods known in the art. In addition, collagen is not limited to any of those which can be classified into types I, II, III, and so on. Of these, however, the type I is particularly preferable in terms of handling. Also, any combination between the origin of the nonwoven fabric and the origin of the filmy material is allowable.

The filmy materials may be in the form of a sheet or film or may be in the form of a sponge having multiple pores, but are not particularly limited to these. A biodegradable nonwoven fabric or the like may be used, but particularly preferable is a biodegradable nonwoven fabric which has been subjected to a binder treatment with a biodegradable material as described above.

The position of a filmy material to be superimposed is not particularly limited. In other words, it may be placed on or under a biodegradable nonwoven fabric. The filmy material may be placed between the biodegradable nonwoven fabrics and the number of laminated layers is not limited. Furthermore, by adjusting the tension of a stitching thread and the number of the laminated nonwoven fabrics, it is possible to control the void fraction.

In this way, the biodegradable substrate of the present invention includes a biodegradable nonwoven fabric which has been stitched with a biodegradable thread. The thread does not require the strength required in a loom or a knitting machine, so that a biodegradable substrate having properties, particularly strength and thickness, similar to woven fabrics can be obtained in an extremely simple manner. The nonwoven fabric is only stitched with the thread. Therefore, there involves no technical difficulty and no use of a special machine such as a loom or knitting machine. It can be prepared using only a simple machine such as a sewing machine. Besides, the threads and fibers, which constitute the nonwoven fabric, are previously bonded to one another. Therefore, a nonwoven fabric which is stable in strength can be simply produced as the thread is hardly loosened when the nonwoven fabric is cut. Since the nonwoven fabric is only subjected to the stitching, the invasion of cells into the substrate and material permeability are not affected by the nonwoven fabric, and it is possible to culture cells in three dimensions.

In particular, in case where a nonwoven fabric including a laminated product in which a first layer and a second layer where a plurality of threads are arranged in parallel are piled on one another is used, the laminated threads can be fixed by stitching threads. The first and second layers in which threads are arranged in parallel are stitched as if the first layer and the second layer are provided as the warp threads and the weft. Therefore, the threads that constitute the nonwoven fabric can be prevented from loosing, and thus it is rare to cause a decrease in strength even if it is cut into any form. Besides, for piling a large number of the first and second layers, there is a need of winding up a very long thread repeatedly into many layers. However, according to the present invention, a substrate having a larger thickness can be obtained by piling many laminated products in which each of the laminated products has a smaller number of layers. Furthermore, the nonwoven fabric has an extremely uniform thickness and provides a substrate having excellent uniformity, compared with one prepared by piling and weaving nonwoven fabrics obtained by a wet paper making method. The thickness or strength of the nonwoven fabric can be comparatively easily and freely adjusted just like a woven fabric or a knitted fabric by adjusting the tension of the stitching thread.

In the case of piling up a filmy material on the nonwoven fabric, there is no specific method required to join the nonwoven fabric and the filmy material. Besides, both of them are stitched together with a biodegradable thread and thus are hardly loosened. Thus, a substrate also having stable quality can be prepared by a simple method.

The prosthetic material for tissue regeneration of the present invention includes the biodegradable substrate (stitched product) obtained as described above. In other words, the biodegradable substrate can be used as a prosthetic material for tissue regeneration (substrate for transplantation), which is transplanted into the living body as filling and prosthetic materials for tissue regeneration, for example, in the field of tissue engineering and regenerative medicine. That is, the biodegradable substrate of the present invention is provided to fill a tissue-defective portion in the living body or on the surface of the body to facilitate the regeneration of the lost biological tissue. Specifically, for example, it can be directly applied as a filmy material for the pericardium, pleura, cerebral dura mater, chorion, and the like to a membrane site removed by a surgical operation. The biodegradable substrate gradually decomposes and is absorbed as the filmy material covering the biological tissue regenerates. Also, as a prosthetic material, it may fill a hole after the extraction of tooth or a hole opened in dental bone to seal the hole until the regeneration of gingival tissue or dental bone. It may be processed into a tubular article such as an artificial blood vessel, a stent, an artificial nerve channel, an artificial trachea, an artificial esophagus, or an artificial ureter, or bag-like articles to be embedded in the living body. As a method of preparing the tubular article, there may be mentioned a method by which the biodegradable substrate obtained is wound around a tube or the like made of polyfluoroethylene fibers using a collagen solution as adhesives, and is dried, followed by pulling the tube out.

Furthermore, for preparing a three-dimensional structure, for example, there may be mentioned a method by which a thick substrate is directly cut and processed, or the substrate obtained is placed in a mold, followed by pouring a biodegradable polymer solution in the mold through a hole formed therein and drying using various methods.

The cultured tissue of the present invention is characterized in that biological tissue cells are adhered, while maintaining their function, to the biodegradable substrate of the present invention. In other words, the biodegradable substrate of the present invention can be also used for a substrate (cell-culture substrate) for in vitro culture of various cells such as adhesive cells. Specifically, the above biodegradable substrate is shaped into a predetermined form and then cells that form body tissues, such as fibroblasts and chondrocytes, are previously cultivated on a culture substrate for a predetermined period according to a conventional method, and then, the cells may be grown into the shape of the culture substrate to form a biological tissue, followed by transplantion into the body. Here, the term "biological tissue" means a tissue whose cells can be cultured in vi tro and includes all tissues which can be cultured, such as cardiac muscles, blood vessels, and skins. For example, cardiac muscle cells may be cultured on the biodegradable substrate and then transplanted together with the biodegradable substrate. After shaping the biodegradable substrate into a tubular material, vascular endothelial cells or epithelial cells may be cultured there and then transplanted together with the biodegradable substrate. After the transplantation, the substrate can be naturally degraded and removed. There is no need for any subsequent operation to remove the substrate.

Furthermore, the biodegradable substrate can be impregnated with various growth factors, medicaments, vectors, and the like so as to be used as drug-delivery system carriers, sustained-release agent carriers, gene therapy carriers, and the like.

Hereinafter, the present invention will be described in more detail with reference to examples. However, the scope of the present invention will not be limited to the examples described below.

### Example 1 Production of collagen nonwoven fabric

Collagen type I and type III powders from a pig (manufactured by Nippon Meat Packers, Inc., SOFD type, Lot No. 0102226) were dissolved in injectable distilled water (manufactured by Otsuka Pharmaceutical Co. , Ltd.) and adjusted to 7 wt%. Subsequently, this 7 wt% collagen aqueous solution was filled into a syringe (manufactured by EFD Inc., Disposable Barrels/Pistons, 55 mL) and extruded into an ethanol bath by air pressure through a needle fitted to the syringe. In this case, the syringe was fitted with a needle of Ultra Dispensing Tips available from EFD Inc., (27G, ID: Ø 0.21 mm) . The extruded 7 wt% collagen aqueous solution was dehydrated to become threadlike and then pulled out from the ethanol bath. The collagen thread thus pulled out was immersed at room temperature for approximately 30 seconds in a second ethanol bath completely separate from the above-described ethanol bath for further solidification. Subsequently, the collagen thread pulled out from the second ethanol bath was wound onto a 15 cm-sided and 5 mm-thick plate member rotating at 15 rpm as shown in Fig. 1. A reciprocating mechanism that periodically moves the horizontal position of the collagen thread for uniformly winding up the collagen thread onto the plate member was arranged immediately in front of the plate member, the reciprocating speed of which was set at 1.5 mm/second (the thread was wound up at an interval of approximately 6 mm). A wind-up device was allowed to change the direction of the rotation axis in the plate member in 90-degree angles, every time it wound up the thread 500 times, and 500-time wind-ups were repeated 9 times (total number of wind-ups: 4500, a laminated product having 9 layers in total) to give a collagen wound-up material having layers of the collagen threads on both sides of the plate member. Next, this collagen wound-up material was naturally dried at room temperature for 4 hours and was then cut along its end to give 2 collagen nonwoven fabrics. Thereafter, the obtained collagen nonwoven fabrics were subj ected to thermal dehydration crosslinking reaction under reduced pressure (1 Torr or less) at 135°C for 24 hours using a vacuum dry oven (manufactured by EYELA Inc. : VOS-300VD type) and an oil-sealed rotary vacuum pump (manufactured by ULVAC KIKO Inc. : GCD135-XA type). The resultant had a thickness of approximately 3 mm.

### Example 2 Secondary process to collagen filmy material

Collagen type I and type III mixed powders from a pig (manufacturedbyNipponMeat Packers, Inc., SOFD type, Lot No. 010226) were dissolved in injectable distilled water (manufacturedbyOtsuka Pharmaceutical Co., Ltd.) and adjusted to 1 wt%. In this collagen aqueous solution adjusted to 1 wt% was immersed one of the collagen nonwoven fabrics after thermal dehydration crosslinking reaction obtained in Example 1, followed by forming into a film and sufficient air-drying at room temperature. Then, the same vacuum dry oven as mentioned above was used to carry out thermal dehydration crosslinking reaction under reduced pressure (1 Torr or less) at 135°C for 12 hours to give a filmy collagen nonwoven fabric.

### Example 3 Production of collagen single thread

As described in Example 1, a 7 wt% collagen aqueous solution was prepared. Then, after the whole spinning environment described below was kept at a relative humidity of 38% or less, a syringe (manufactured by EFD Inc., Disposable Barrels/Pistons, 55 mL) filled with the 7 wt% collagen aqueous solution was allowed to receive air pressure to extrude the collagen aqueous solution through a needle fitted to the syringe. In this case, the syringe was fitted with a needle of Ultra Dispensing Tips available from EFD Inc., (27G, ID: 0.21 mm). The extruded 7 wt% collagen aqueous solution was immediately dehydrated and solidified into a thread shape in an ethanol bath containing 3 L of 99.5 vol% ethanol (Wako Pure Chemical Industries, Ltd., special grade). The threadlike collagen pulled out from the ethanol bath was immersed at room temperature for approximately 30 seconds in a second ethanol bath containing 3 L of 99.5 vol% ethanol (Wako Pure Chemical Industries, special grade), which is completely separate from the above-described ethanol bath, for additional dehydration and solidification. Subsequently, the threadlike collagen pulled out from the second ethanol bath was passed through an air dryer supplying dry air into its surroundings in 3 seconds and was then wound up onto a roll-like wind-up tool made of stainless steel with a diameter of 78 mm and an overall length of 200 mm which was allowed to rotate at 35 rpm, while the tension was maintained with a tension pulley to prevent the thread from loosing. This roll-like wind-up tool wound up the threadlike collagen while reciprocating it at a speed of 1.5 mm/s in the axis direction of the roll-like wind-up tool and carried out continuous spinning until the 7 wt% collagen aqueous solution filled into the syringe ran out. In this manner, a bobbin of collagen single thread was obtained.

### Example 4 Thermal dehydration and crosslinking reaction treatment of collagen single thread

The collagen single thread produced in Example 3 was subjected to thermal dehydration and crosslinking reaction treatment in the state of being wound up in a roll-like wind-up tool made of stainless steel under reduced pressure (1 Torr or less) at 135°C for 24 hours using the same vacuum dry oven as described above to give a bobbin of collagen single thread treated with thermal crosslinking.

### Example 5 Stitching of collagen nonwoven fabric

Six collagen nonwoven fabrics as produced in Example 1 were piled on one another and stitched with a two-ply thread twisted with the collagen single thread as produced in Example 3 to give a three-dimensional biodegradable substrate having a collagen thread structure. A sewing machine (manufactured by Jaguar Inc., Model KM-570) was used for stitching. Sewing patterns were composed of straight stitches having a pitch of 2 to 3 mm and the fabric was stitched lengthwise and crosswise (at an interval of approximately 1 cm) around the collagen nonwoven fabric and over the nonwoven fabric.

### Example 6 Stitching of collagen nonwoven fabric and collagen filmy material

The collagen nonwoven fabric as produced in Example 1 was piled on the collagen nonwoven fabric formed into a film as produced in Example 2 , and stitched with a thermal-crosslinked two-ply thread twisted with the collagen single thread as produced in Example 4 to give a three-dimensional biodegradable substrate having a collagen thread structure. A stitching method was the same as that in Example 5.

### Experimental Example 1 Fibroblast culture experiment

Human fibroblast culture was carried out on the substrate made of collagen which was obtained in Example 5. For culture, there was used a mixed medium in which 500 mL of Medium 106S (basal medium) and 10 mL of LSGS (Low Serum Growthfactor Supplement) (both are manufactured by Cascade Biologics, Inc.) were mixed.

At first, the collagen nonwoven fabric was left to stand in a petri dish (manufactured by CORNING Inc., 6 wells) and 1 mL of the above-described mixed medium in which the cells were suspended to have a concentration of 4.0 x 10⁵ cells/mL was applied onto the substrate. Then, 3 mL of the medium was gently poured into the petri dish and left to stand for culture under the culture conditions of a temperature of 37°C and a CO₂ concentration of 5%.

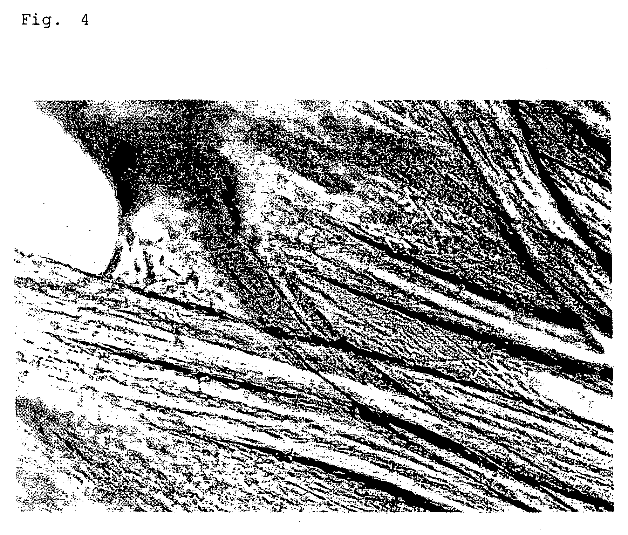

Fig. 4 shows the state of the adhesion of the cells, while maintaining their function, to the substrate and the amplification of the cell after 14 days from the initiation of culture. As a result, it was confirmed that the cells firmly adhered, while maintaining their function, to the collagen threads arranged lengthwise and crosswise. It was also confirmed that the cells were well amplified along the collagen thread constituting the substrate. This indicates that the substrate can sufficiently function as a three-dimensional culture substrate made of collagen according to the present invention.

### Experimental Example 2 Chondrocyte culture experiment

Human chondrocyte culture was carried out on the substrate made of collagen which was obtained in Example 5. For culture, there was used a mixed medium in which 500 mL of basal medium and 10 mL of Growth Supplement (both are manufactured by CELL APPLICATIONS, Inc.) were mixed.

At first, the collagen nonwoven fabric was left to stand in a petri dish (manufactured by CORNING Inc., 6 wells) and 1 mL of the above-described mixed medium in which the cells were suspended to have a concentration of 4.0 x 10⁵ cells/mL was applied onto the substrate. Then, 3 mL of the medium was gently poured into the petri dish and left to stand for culture under the culture conditions of a temperature of 37°C and a CO₂ concentration of 5%.

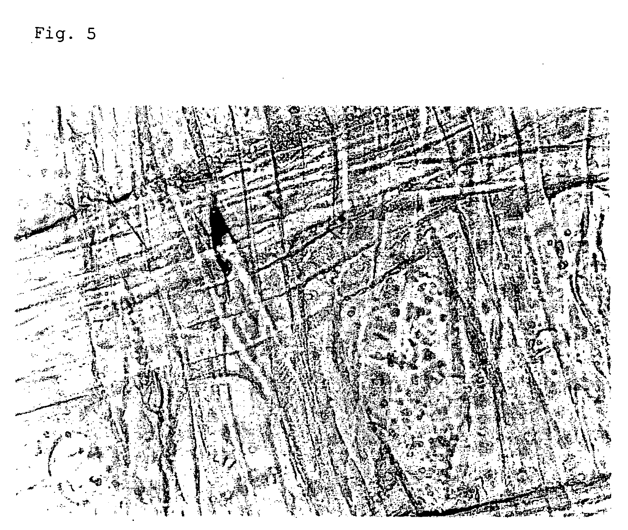

Fig. 5 shows the state of the adhesion of the cells, while maintaining their function, to the substrate and the amplification of the cells after 14 days from the initiation of culture. As a result, it was confirmed that the cells firmly adhered, while maintaining their function, to the collagen threads arranged lengthwise and crosswise. It was also confirmed that the cells were well amplified along the collagen thread constituting the substrate. This demonstrates that the substrate can sufficiently function as a three-dimensional culture substrate made of collagen according to the present invention.

### INDUSTRIAL APPLICABILITY

The biodegradable substrate of the present invention includes a biodegradable nonwoven fabric which is stitched with a biodegradable thread. Thus, a nonwoven fabric having excellent affinity to cells and given thickness and strength can be easily obtained from a thin nonwoven fabric having insufficient strength by a simple apparatus such as a sewing machine. In addition, a biodegradable thread used for the nonwoven fabric or a biodegradable thread used for stitching requires strength sufficient for the fabric to be wound up but does not require strength necessary for the fabric to be used in a loom or a knitting machine. Therefore, a biodegradable thread obtained by any method conventionally known in the art can be directly used.

In particular, a biodegradable thread such as a collagen thread is hardly imparted with high strength. However, it is possible to produce a biodegradable substrate having properties comparable with those of a woven or knitted fabric, for example, sufficient strength and thickness even from such collagen threads. Besides, the cut biodegradable fabric is rare to loosen the warp and the weft unlike woven fabrics, and thus can be molded into any desired shape. Further, the thickness and strength can be relatively freely adjusted, whereby the substrate enabling the three-dimensional cell culture can be obtained with ease and at low cost.

## Claims

1. A biodegradable substrate, which comprises a biodegradable nonwoven fabric that is stitched with a biodegradable thread.

2. A biodegradable substrate, which comprises a biodegradable nonwoven fabric and a biodegradable filmy material that are piled up on one another and stitched with a biodegradable thread.

3. The biodegradable substrate according to claim 2, wherein the biodegradable filmy material is in the form of a film or a sponge.

4. The biodegradable substrate according to any one of claims 1 to 3, wherein the biodegradable nonwoven fabric comprises a laminated product including a first layer and a second layer, each of which has a plurality of biodegradable threads arranged in parallel, the first layer and the second layer being piled up on one another such that an arranging direction of the threads of the first layer and an arranging direction of the threads of the second layer are at an angle with respect to each other, and adhered to each other.

5. The biodegradable substrate according to claim 4, wherein the laminated product further includes a third layer having a plurality of biodegradable threads arranged in parallel on the first layer or the second layer, where an arranging direction of the threads of the third layer and an arranging direction of the threads of the layer adjacent to the third layer are at an angle with respect to each other, and the third layer and its adjacent layer are adhered to each other.

6. The biodegradable substrate according to claim 4 or 5, wherein an acute angle between the arranging direction of the threads of the first layer and the arranging direction of the thread of the second layer, and/or an acute angle between an arranging direction of the threads of the third layer and an arranging direction of the threads of the layer adjacent to the third layer are/is about 20° or less.

7. The biodegradable substrate according to claim 4 or 5, wherein an acute angle between the arranging direction of the threads of the first layer and the arranging direction of the thread of the second layer, and/or an acute angle between an arranging direction of the threads of the third layer and an arranging direction of the threads of the layer adjacent to the third layer are/is about 70° to 90°.

8. The biodegradable substrate according to any one of claims 4 to 7, wherein the biodegradable nonwoven fabric comprises a plurality of the laminated products, each of which contains a plurality of layers having threads arranged therein and piled up on one another, and the laminated products are piled up one another and adhered to each other such that arranging directions of the threads of the layers adjacent to each other at an interface between the laminated products are at an angle with respect to each other.

9. The biodegradable substrate according to any one of claims 4 to 8, wherein the threads arranged on a respective layer have an acute angle of about 0 to 5° therebetween.

10. The biodegradable substrate according to any one of claims 4 to 9, wherein the threads in each layer are arranged at intervals of about 0 to 40 mm.

11. The biodegradable substrate according to any one of claims 1 to 10, **characterized in that** stitches are made in a dotted pattern.

12. The biodegradable substrate according to claim 11, wherein the stitches are made at intervals of about 0.1 mm to 100 mm.

13. The biodegradable substrate according to any one of claims 1 to 12, wherein the surface of the biodegradable nonwoven fabric and/or the surface of the biodegradable thread are/is coated with a biodegradable material.

14. The biodegradable substrate according to claim 13, wherein the biodegradable material is comprised of one or more materials selected from the group consisting of collagen, gelatin, PLA, PLAderivatives, PGA, PGA derivatives and copolymers formed of two or more of PLA, PLA derivatives, PGA and PGA derivatives.

15. The biodegradable substrate according to any one of claims 1 to 14, **characterized in that** the biodegradable nonwoven fabric and/or the biodegradable thread are/is comprised of one or more materials selected from the group consisting of collagen, gelatin, PLA, PLA derivatives, PGA, PGA derivatives and copolymers formed of two or more of PLA, PLA derivatives, PGA and PGA derivatives.

16. A prosthetic material for tissue regeneration, which is filled in a defective portion in a biological tissue, comprising a biodegradable substrate according to any one of claims 1 to 15.

17. A cultured tissue, **characterized in that** biological tissue cells are adhered to a biodegradable substrate according to any one of claims 1 to 15.
